# EUROPEAN PATENT SPECIFICATION

(11) **EP 3 551 205 B1**
(45) Date of publication and mention of the grant of the patent: **23.07.2025**
(21) Application number: 17818284.6
(22) Date of filing: 29.11.2017
(51) Int. Cl.: A61K 36/63

(54) **OLEA EUROPAEA POMACE EXTRACT AND METHOD FOR PRODUCING THE EXTRACT**
EXTRAKT AUS OLEA-EUROPAEA-TRESTER UND VERFAHREN ZUR HERSTELLUNG DES EXTRAKTS
EXTRAIT DE MARC D'OLEA EUROPAEA ET PROCÉDÉ DE PRODUCTION DUDIT EXTRAIT

(30) Priority: 12.12.2016 ZA 201608578
(43) Date of publication of application: 16.10.2019
(73) Proprietor: Nelson Mandela University, 6001 Port Elizabeth (ZA)
(72) Inventor: VORSTER, Nicole Madeleine, 6001 Port Elizabeth (ZA); POSTMA-BOTHA, Martha Leona, 6001 Port Elizabeth (ZA)
(74) Representative: Hoffmann Eitle
(86) International application number: PCT/IB2017/057488
(87) International publication number: WO 2018/109600

(56) References cited:
- WO-A1-2008/142178
- ES-A1- 2 311 401
- "Modern Extraction Techniques : Food and Agricultural Samples", vol. 926, 6 January 2006, AMERICAN CHEMICAL SOCIETY, Washington, DC, ISBN: 978-0-8412-2044-7, article MONICA WALDEBÄCK ET AL: "Pressurized Fluid Extraction of Squalene from Olive Biomass : Food and Agricultural Samples", pages: 96 - 106, XP055446486, DOI: 10.1021/bk-2006-0926.ch007
- NADIA MULINACCI ET AL: "Solid Olive Residues: Insight into Their Phenolic Composition", JOURNAL OF AGRICULTURAL AND FOOD CHEMISTRY, vol. 53, no. 23, 1 November 2005 (2005-11-01), pages 8963 - 8969, XP055446445, ISSN: 0021-8561, DOI: 10.1021/jf051398r
- KALOGERAKIS NICOLAS ET AL: "Recovery of antioxidants from olive mill wastewaters: A viable solution that promotes their overall sustainable management", JOURNAL OF ENVIRONMENTAL MANAGEMENT, vol. 128, 12 July 2013 (2013-07-12), pages 749 - 758, XP028706984, ISSN: 0301-4797, DOI: 10.1016/J.JENVMAN.2013.06.027

## Description

### INTRODUCTION

This invention relates to an *Olea europaea* pomace extract comprising water-soluble and oil-soluble compounds, and a single step extraction method to produce the extract. The invention further relates to a formulation comprising the extract , e.g., pharmaceutical, cosmetic or food formulations.

### BACKGROUND

During the production of oil from the fruit (olive) of *Olea europaea,* waste material, or pomace, is generated which includes solid waste and/or liquid waste depending on the technique implemented. In a typical modern two phase olive oil processing technique a solid-liquid mill waste is generated consisting of skin, seed, pulp, stone pieces and vegetation liquid.

The aforementioned processing by-product represents a major disposal challenge due to its high organic content. However, this waste material is a potential source of high-value biological compounds for use in, for example, the pharmaceutical, cosmetic and food industries.

Polyphenols are water-soluble antioxidants and the high content of these in the waste pomace presents a major disposal challenge due to the high biological and chemical oxygen demand (BOD and COD) of the waste material. The pomace must first be left to degrade the antioxidant content before it can be used as fertilizer for the soil. This presents a problem of foul odour, attracting mosquitos and bacteria and causing a potential health hazard. If disposed of directly, pollution of both the soil and natural water supply will occur as antioxidants are toxic to soil and water organisms. Disposal costs for oil processing plants are thus high as the waste needs to be treated significantly to degrade phenolics before disposal.

Leaf extracts from *Olea europaea, i.e.* "olive leaf', are well-known in the health supplement market and are offered as tinctures or in capsule form for health and anti-aging benefits. The main ingredient in olive leaf is a water-soluble antioxidant called oleuropein. However, olive leaf extracts rely on a supply of *Olea europaea* leaves, and are therefore less sustainable than would be extracts produced from pomace.

WO 2008/142178 A1 relates to a method for extraction of compounds with antioxidant capacity from the products derived from olive oil production. The starting material for the method can be selected from either vegetation waters of the oil production process, or a concentrated extract of these vegetation waters.

M. Waldebäck et al. describe in "Pressurized Fluid Extraction of Squalene from Olive Biomass" in "Modern Extraction Techniques: Food and Agricultural Samples" of the ACS Symposium Series of the American Chemical Society in vol. 926, pp. 96-106 (January 6, 2006) a study using pressurized fluid extraction (PFE) technique to extract the oil-soluble compounds squalene and α-tocopherol from olive oil pomace.

N. Mulinacci et al. describe in Journal of Agricultural and Food Chemistry 2005, vol. 53, pp. 8963-8969 methods of extracting phenolics from solid olive residues, which are byproducts of the olive-milling process. Hydroalcoholic extraction was applied.

N. Kalogerakis et al. in Journal of Environmental Management 2013, vol. 128, pp. 749-758 describe the recovery of antioxidants from olive mill wastewaters. A liquidliquid solvent extraction was employed to recover various polyphenolic compounds such as hydroxytyrosol and tyrosol.

There is therefore a need for an extract produced from pomace to address the issue of waste generated during olive oil production, for an extract that comprises water-soluble and oil-soluble bioactive ingredients, and for an extraction method to produce the extract.

### SUMMARY OF THE INVENTION

The present invention t provides a method of producing an extract from pomace obtained from an *Olea europaea* fruit oil production process as defined in independent claim 1. Furthermore, the present invention provides an extract obtainable by the above production method that is claimed in independent claim 10. Finally, the present invention provides a formulation comprising the extract, which is the subject of independent claim 14. Preferred embodiments of the method, extract and formulation according to the present invention are the subject of the dependent claims.

### BRIEF DESCRIPTION OF THE DRAWINGS

The invention will now be described in more detail with reference to the following embodiments and figures in which:
- **Figure 1**: shows a representation of a modified Soxhlet extraction setup used in the method of the present invention;
- **Figure 2**: shows an HPLC chromatogram of the water-soluble compounds in a sample of the extract of the present invention;
- **Figure 3**: shows an HPLC chromatogram of a reference standard sample for the water-soluble compounds hydroxytyrosol, tyrosol, and oleuropein;
- **Figure 4**: shows an HPLC chromatogram of the oil-soluble compounds in a sample of the extract of the present invention; and
- **Figure 5**: shows an HPLC chromatogram of a reference standard sample for the oil-soluble compounds alpha-tocopherol and squalene.

### DETAILED DESCRIPTION OF A PREFERRED EMBODIMENT

The present invention will now be described more fully hereinafter with reference to the accompanying figures, in which some of the embodiments of the invention are shown.

The invention as described hereinafter should not be construed to be limited to the specific embodiments disclosed, with slight modifications and other embodiments intended to be included within the scope of the invention as defined in the claims. Although specific terms are employed herein, they are used in a generic and descriptive sense only.

As used herein, throughout this specification and in the claims which follow, the singular forms "a", "an" and "the" include the plural form, unless the context clearly indicates otherwise.

The terminology and phraseology used herein is for the purpose of description. The use of the terms "comprising", "containing" "having", "including", and variations thereof used herein, are meant to encompass the items listed thereafter, and equivalents thereof as well as additional items.

The present invention relates to an extract as further defined in the claims produced from the waste material (pomace) that is produced as a by-product of the oil extraction process from the fruit of *Olea europaea,* wherein the extract comprises water-soluble and oil-soluble compounds. The extract is produced in a single step extraction process that utilises an extraction solvent mixture comprising at least three constituents, namely, an alcohol; and an organic component selected from n-heptane and ethyl acetate; and water. The extraction solvent mixture optionally further comprises limonene.

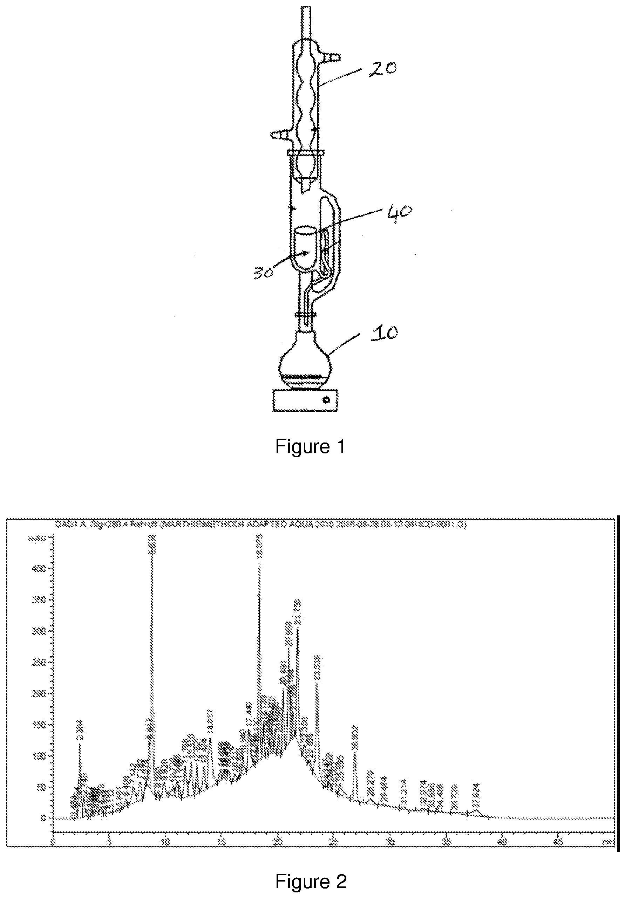

In one embodiment of the invention, the method is a modified Soxhlet type extraction method. With reference to Figure 1, a modified Soxhlet extraction method was used in which a vacuum pump was fitted to the system to reduce the pressure in the closed system in order for the solvents to be refluxed at a temperature lower than the boiling point of the solvent mixture at atmospheric pressure.

In one embodiment a vacuum pump was connected to the top of the condenser 20 to reduce the pressure, thereby allowing the solvent blend to reflux at a lower temperature. Preferably the solvent blend is refluxed at a temperature of about 40 °C - 70 °C, more preferably about 45 °C - 65 °C, even more preferably about 60 °C. The condenser coils may be cooled with chilled water at about 3 - 5 °C using a chiller unit with a circulating pump.

The pressure is reduced in the system so that the extraction solvent mixture can be refluxed at a lower temperature to provide for and accommodate the temperature sensitivity of the target compounds. In one embodiment the pressure may be reduced to about 150 - 450 mbar, preferably the pressure is reduced to about 250 - 400 mbar, most preferably the pressure is reduced to about 350 mbar.

Soxhlet extraction is based on the principle of a continuous extraction system in which a solvent or solvent blend is heated and stirred in a round-bottomed flask 10 with constant stirring until the solvent or blend boils (refluxes). This solvent is then condensed using a cooling system 20 and the condensed solvent then drips onto and diffuses through the pomace starting material 30 (contained in a cellulose thimble) out of which the desired compounds are to be extracted. The pomace starting material may be extracted as is, i.e. in the solid-liquid state as obtained from the olive oil extraction process, or it may be dried prior to extraction, for example by freeze drying.

Referring again to Figure 1, as the glass unit 40 in which the thimble is contained is filled to the top the solvent siphons back into the round bottom flask 10 below taking with it the extracted compounds which are dissolved in the solvent or solvent blend. The extraction process continues as the solvent is refluxed. After a specified period of time, the extraction is stopped, the heat removed and the solvents (now containing the active compounds), which may form a two-phase system depending on the selected solvents or solvent mixture within the scope of the appended claims, are separated in a separating funnel if necessary, and evaporated under vacuum in a rotary evaporator leaving behind the extracted residues.

It should however be understood that the method of invention is not limited to a Soxhlet type extraction as shown in Figure 1. The method according to the present invention provides primarily for an extraction solvent mixture within the scope of the appended claims that is directed at extracting both water-soluble and oil-soluble biologically active compounds from pomace, in a single step extraction method.

The solvent blend used in the method of the invention to extract both water-soluble and oil-soluble biologically active compounds from pomace optionally comprises up to about 30 vol. % of the organic component, from about 0 - 20 vol. % of limonene, from about 50 - 100 vol. % alcohol, and up to about 50 vol. % water. The organic component is n-heptane or ethyl acetate, and the alcohol is preferably methanol, ethanol, propanol. Most preferably, the alcohol is ethanol.

Surprisingly, it was found that even though the solubility parameter of ethanol most closely matched the average solubility parameter of the water-soluble and oil-soluble biologically active compounds, ethanol alone was not able to extract all the desired bioactives. The presence of a high polarity solvent like water and a very nonpolar organic solvent like heptane enhanced the extraction capability of the ethanol.

In a further aspect of the invention it is foreseen that specific target active compounds in the extract can be increased or decreased by modification of the solvent ratios in the solvent blend used in the extraction method.

The method may therefore further provide for in-process active compound concentration monitoring, with the solvent blend adjusted accordingly to achieve the optimum active compound concentrations in the final extract. For example, the ratios of the various solvents in the solvent blend may be adjusted according to Table 1 below to target specific compounds during the extraction process.

**Table 1: Predicted maximum responses and solvent blends for each bioactive compound during combination extractions of water- and lipid-soluble compounds from Frantoio pomace.**

| | **Vol %** | | | | **mg/g dry weight pomace starting material** | | | | | **hours** |
|---|---|---|---|---|---|---|---|---|---|---|
| | **n-Hep** | **Lim** | **EtOH** | **Water** | **Htyr** | **Tyr** | **Oleu** | **α-Toco** | **Squal** | **Time** |
| Max HTyr | 0 | 0 | 68.5 | 31.5 | 1.62 | 0.15 | 0.24 | 0.05 | 0.06 | 5 |
| Max Tyr | 0 | 0 | 100 | 0 | 1.35 | 0.16 | 0.16 | 0.03 | 0.14 | 5 |
| Max Oleu | 30 | 0 | 50 | 20 | 1.38 | 0.14 | 0.25 | 0.05 | 0.62 | 2 |
| Max a-toco | 30 | 0 | 50 | 20 | 0.83 | 0.10 | 0.11 | 0.08 | 0.34 | 5 |
| Max squal | 0 | 20 | 80 | 0 | 0.44 | 0.1 | 0.05 | 0.00 | 0.66 | 5 |
| Opt blend | 30 | 0 | 50 | 20 | 1.38 | 0.14 | 0.25 | 0.05 | 0.62 | 2 |

| | | | | | | | | | | |
|---|---|---|---|---|---|---|---|---|---|---|
| *n*-Hep: *n*-Heptane, Lim: Limonene, EtOH: Ethanol, SP: Solubility parameter, HTyr: Hydroxytyrosol , Tyr: Tyrosol, Oleu: Oleuropein, α-Toco: α-Tocopherol, and Squal: Squalene. | | | | | | | | | | |

Furthermore, the method may be applied to different olive cultivars to obtain the average amounts of the selected bioactive compounds as shown in Table 2 below.

**Table 2: Average content (mg/g dry pomace starting material) of each bioactive compound in two different olive cultivars from the combined extraction using a solvent blend of n-heptane/ethanol/water of 30/50/20 vol % at 350 mbar and ± 60 °C reflux temperature (2 hours extraction time).**

| **Bioactive compound** | ***Coratina*** | ***Frantoio*** |
|---|---|---|
| | **Ave content (mg/g DW)** | **Ave content (mg/g DW)** |
| Hydroxytyrosol | 0.240 | 0.482 |
| Tyrosol | 0.153 | 0.237 |
| Oleuropein | 3.495 | 0.258 |
| α-Tocopherol | 0.061 | 0.033 |
| Squalene | 0.753 | 0.464 |

The concentration of the various active compounds in the extract may be quantified by standard methods known in the art such as high performance liquid chromatography (HPLC), or HPLC coupled with other analytical methods such as mass spectroscopy.

In one example an extraction was performed using the extraction configuration provided in Figure 1. The pressure in the system was reduced to 350 mbar and the reflux temperature set at 60 °C.

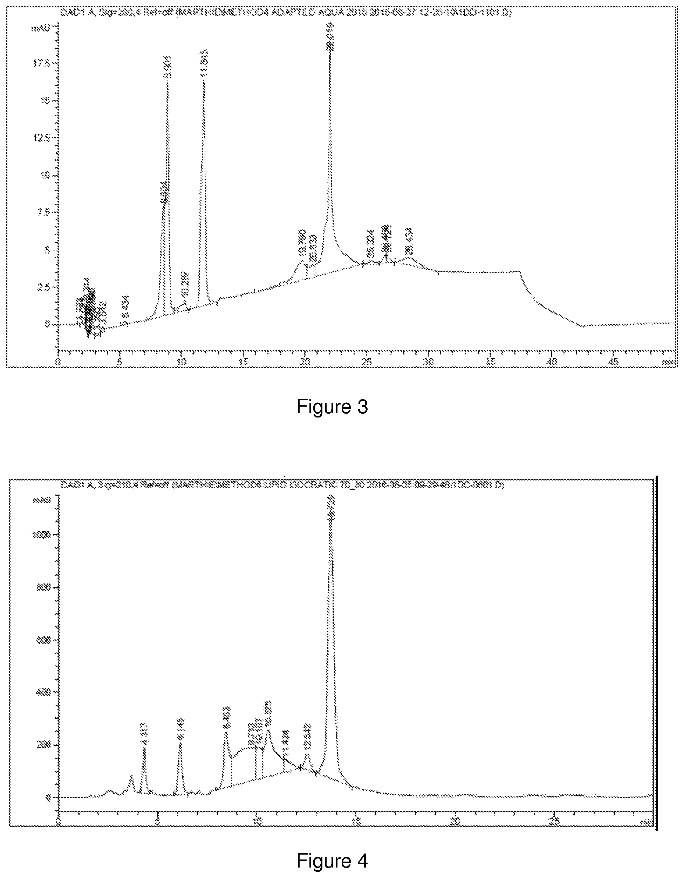

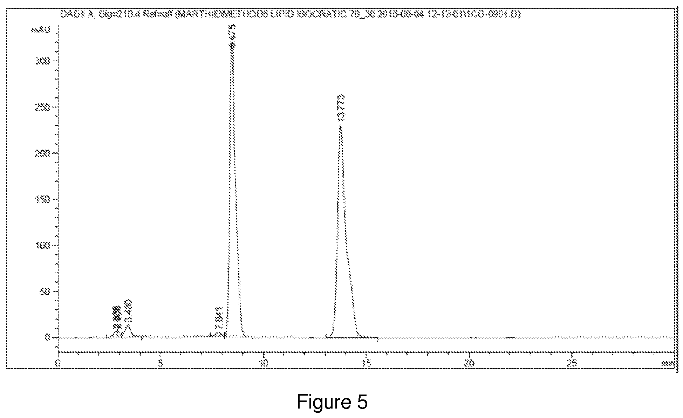

A solvent blend comprising 30 % n-heptane, 50 % ethanol, and 20 % water was added to the round bottom flask 10 and refluxed for 2 hours. The round bottom flask was removed, the phases separated and the extracts dried under vacuum in a rotary evaporator. The extracts were reconstituted and samples were prepared for HPLC testing against standard solutions. Figures 2 and 3 show HPLC chromatograms of sample solutions and reference standard solutions for the water-soluble compounds (hydroxytyrosol, tyrosol, and oleuropein), while Figure 4 and 5 show HPLC chromatograms of sample solutions and reference standard solutions for oil-soluble compounds (α-tocopherol and squalene).

Within the scope of the appended claims, the solvent blend may be adjusted and optimised for water-soluble antioxidants or oil-soluble compounds having moisturising properties depending on the application for which the extract is intended, for example for skin care products or health supplement products.

The extracts can be formulated into cosmetic skincare products with cosmeceutical properties e.g. antioxidant, anti-aging, skin-healing and treatment of problem skin, or encapsulated into a soft gel to be used as a health supplement. Additionally, depending on the application, the pomace extract can be provided in a liquid form or freeze-dried and processed into a powder which can be used in a number of food applications.

The extracts of the present invention may be formulated into cosmetic products for the treatment of dry skin, dermatitis and eczema. The cosmetic products may be formulated as ointments, creams, lotions, gels, foot scrubs, or hair products such as shampoos or waxes.

The extracts of the present invention may be formulated into pharmaceutical formulations or complementary health products. These products may be provided as tablets, caplets, capsules, soft gel capsules, elixirs, syrups, or creams.

In addition, the extracts of the present invention may also be formulated into food products including sauces and spices, and dairy products such as butter and yoghurt.

This above description of some of the illustrative embodiments of the invention is to indicate how the invention can be made and carried out. Those of ordinary skill in the art will know that various details may be modified thereby arriving at further embodiments, but that many of these embodiments will remain within the scope of the invention as defined in the claims.

## Claims

1. A method of producing an extract from pomace obtained from an Olea *europaea* fruit oil production process, the method comprising the step of:
providing pomace starting material including mixtures of fruit skin, seed, pulp, and vegetation liquid;
providing an extraction solvent mixture comprising:
a) an organic solvent component selected from n-heptane and ethyl acetate, and
b) alcohol, and
c) water;
extracting the starting material with the solvent mixture in a single step extraction to produce an extract comprising water-soluble and oil-soluble compounds including hydroxytyrosol, tyrosol, oleuropein, α-tocopherol, and squalene.

2. The method according to claim 1, wherein the solvent mixture further comprises limonene.

3. The method according to claim 1 or 2, wherein the solvent mixture comprises about 2 - 50 vol. % of the organic component, about 0 - 30 vol. % of limonene, about 30 - 90 vol. % alcohol, and about 2 - 50 vol. % water.

4. The method according to claim 3, wherein the solvent mixture comprises about 30 vol. % of the organic component, about 50 vol. % alcohol, and about 20 vol. % water.

5. The method according to any one of claims 1 to 4, wherein the alcohol is selected from the group consisting of methanol, ethanol, and isopropanol.

6. The method of claim 5, wherein the alcohol is ethanol.

7. The method according to any one of claims 1 to 6, wherein the method is a modified Soxhlet extraction method in which the pressure is reduced to about 150 - 450 mbar.

8. The method according to any one of claims 1 to 7, wherein the extraction temperature is about 40 °C - 60 °C.

9. The method according to any one of claims 1 to 8, wherein the pomace starting material is extracted for about 2 - 5 hours.

10. An extract obtainable by the production method according to any one of claims 1 to 9.

11. The extract of claim 10, wherein the ratios of hydroxytyrosol, tyrosol, oleuropein, and squalene to α-tocopherol are hydroxytyrosol:α-tocopherol is greater than 3.0:1, tyrosol:α-tocopherol is greater than 2.0:1, oleuropein:α-tocopherol is greater than 2.0:1, and squalene:α-tocopherol is greater than 10:1.

12. The extract of claim 10 or 11, wherein the extract comprises a mixture of about 0.24 - 1.65 mg/g hydroxytyrosol, about 0.10 - 0.14 mg/g tyrosol, about 0.05 - 3.5 mg/g oleuropein, about 0.01 - 0.08 mg/g α-tocopherol, and about 0.06 - 0.753 mg/g squalene, per dry weight of the pomace starting material.

13. The extract of any one of claims 10 to 12, wherein the extract is a crude extract or a purified extract, or wherein the extract is a powder extract or a liquid extract.

14. A formulation comprising an extract according to any one of claims 10 to 13, which is a tablet, capsule, soft-gel capsule, elixir, syrup, cream, lotion, gel, or an ointment.

15. The formulation of claim 14, wherein the formulation is a pharmaceutical formulation, complementary health product formulation, cosmetic formulation, or a food product formulation.

## Patentansprüche

1. Verfahren zur Herstellung eines Extrakts aus Trester, erhalten aus einem Ölherstellungsprozess aus der Olea europaea-Frucht, wobei das Verfahren die folgenden Schritte umfasst:
Bereitstellen eines Trester-Ausgangsmaterials, das Mischungen aus Fruchtschale, Kernen, Fruchtfleisch und Vegetationsflüssigkeit einschließt;
Bereitstellen einer Extraktionslösemittelmischung, die folgendes umfasst:
a) eine organische Lösemittelkomponente, ausgewählt aus n-Heptan und Ethylacetat, und
b) Alkohol und
c) Wasser;
Extrahieren des Ausgangsmaterials mit der Lösemittelmischung in einer einstufigen Extraktion, um so einen Extrakt herzustellen, der wasserlösliche und öllösliche Verbindungen, eingeschlossen Hydroxytyrosol, Tyrosol, Oleuropein, α-Tocopherol und Squalen, umfasst.

2. Verfahren nach Anspruch 1, bei dem die Lösemittelmischung ferner Limonen umfasst.

3. Verfahren nach Anspruch 1 oder 2, bei dem die Lösemittelmischung etwa 2 - 50 Vol.-% der organischen Komponente, etwa 0 - 30 Vol.-% Limonen, etwa 30 - 90 Vol.-% Alkohol und etwa 2 - 50 Vol.-% Wasser umfasst.

4. Verfahren nach Anspruch 3, bei dem die Lösemittelmischung etwa 30 Vol.-% der organischen Komponente, etwa 50 Vol.-% Alkohol und etwa 20 Vol.-% Wasser umfasst.

5. Verfahren gemäß mindestens einem der Ansprüche 1 bis 4, bei dem der Alkohol ausgewählt ist aus der Gruppe, bestehend aus Methanol, Ethanol und Isopropanol.

6. Verfahren nach Anspruch 5, bei dem der Alkohol Ethanol ist.

7. Verfahren gemäß mindestens einem der Ansprüche 1 bis 6, wobei das Verfahren ein modifiziertes Soxhlet-Extraktionsverfahren ist, bei dem der Druck auf etwa 150 - 450 mbar reduziert ist.

8. Verfahren gemäß mindestens einem der Ansprüche 1 bis 7, bei dem die Extraktionstemperatur etwa 40°C - 60°C ist.

9. Verfahren gemäß mindestens einem der Ansprüche 1 bis 8, bei dem das Trester-Ausgangsmaterial für etwa 2 - 5 Stunden extrahiert wird.

10. Extrakt, erhältlich durch das Produktionsverfahren gemäß mindestens einem der Ansprüche 1 bis 9.

11. Extrakt nach Anspruch 10, in dem die Verhältnisse von Hydroxytyrosol, Tyrosol, Oleuropein und Squalen zu α-Tocopherol sind: Hydroxytyrosol:α-Tocopherol ist größer als 3,0:1, Tyrosol:α-Tocopherol ist größer als 2,0:1, Oleuropein:α-Tocopherol ist größer als 2,0:1 und Squalen:α-Tocopherol ist größer als 10:1.

12. Extrakt nach Anspruch 10 oder 11, wobei der Extrakt eine Mischung von etwa 0,24 - 1,65 mg/g Hydroxytyrosol, etwa 0,10 - 0,14 mg/g Tyrosol, etwa 0,05 - 3,5 mg/g Oleuropein, etwa 0,01 - 0,08 mg/g α-Tocopherol und etwa 0,06 - 0,753 mg/g Squalen, pro Trockengewicht des Trester-Ausgangsmaterials, umfasst.

13. Extrakt gemäß mindestens einem der Ansprüche 10 bis 12, wobei der Extrakt ein Rohextrakt oder ein gereinigter Extrakt ist oder bei dem der Extrakt ein Pulverextrakt oder ein Flüssigextrakt ist.

14. Formulierung, die einen Extrakt nach mindestens einem der Ansprüche 10 bis 13 umfasst, die eine Tablette, Kapsel, Weichgelkapsel, ein Elixier, Sirup, eine Creme, Lotion, ein Gel oder eine Salbe ist.

15. Formulierung nach Anspruch 14, wobei die Formulierung eine pharmazeutische Formulierung, Gesundheitsergänzungsprodukt-Formulierung, kosmetische Formulierung oder Lebensmittelformulierung ist.

## Revendications

1. Procédé de production d'un extrait de marc obtenu à partir d'un processus de production d'huile de fruit d'*Olea europaea,* le procédé comprenant l'étape consistant à :
fournir un matériau initial de marc incluant des mélanges de peau de fruit, de graine, de pulpe et de liquide végétal ;
fournir un mélange de solvants d'extraction comprenant :
a) un composant de solvant organique sélectionné à partir du n-heptane et de l'acétate d'éthyle, et
b) de l'alcool, et
c) de l'eau ;
extraire le matériau initial avec le mélange de solvants lors d'une étape unique d'extraction pour produire un extrait comprenant des composés solubles dans l'eau et insolubles dans l'huile incluant l'hydroxytyrosol, le tyrosol, l'oleuropéine, l'α-tocophérol et le squalène.

2. Procédé selon la revendication 1, dans lequel le mélange de solvants comprend en outre du limonène.

3. Procédé selon la revendication 1 ou la revendication 2, dans lequel le mélange de solvants comprend environ 2 à 50 % en volume du composant organique, environ 0 à 30 % en volume de limonène, environ 30 à 90 % en volume d'alcool et environ 2 à 50 % en volume d'eau.

4. Procédé selon la revendication 3, dans lequel le mélange de solvants comprend environ 30 % en volume du composant organique, environ 50 % en volume d'alcool et environ 20 % en volume d'eau.

5. Procédé selon l'une quelconque des revendications 1 à 4, dans lequel l'alcool est sélectionné à partir du groupe constitué du méthanol, de l'éthanol et de l'isopropanol.

6. Procédé selon la revendication 5, dans lequel l'alcool est l'éthanol.

7. Procédé selon l'une quelconque des revendications 1 à 6, dans lequel le procédé est un procédé d'extraction Soxhlet modifiée dans lequel la pression est réduite à environ 150-450 mbar.

8. Procédé selon l'une quelconque des revendications 1 à 7, dans lequel la température d'extraction est d'environ 40 °C - 60 °C.

9. Procédé selon l'une quelconque des revendications 1 à 8, dans lequel la matière initiale à base de marc est extraite pendant environ 2 à 5 heures.

10. Extrait pouvant être obtenu par le procédé de production selon l'une quelconque des revendications 1 à 9.

11. Extrait selon la revendication 10, dans lequel les rapports de l'hydroxytyrosol, du tyrosol, de l'oleuropéine et du squalène à l'α-tocophérol sont hydroxytyrosol/α-tocophérol supérieur à 3,0/1, tyrosol/α-tocophérol supérieur à 2,0/1, oleuropéine/α-tocophérol supérieur à 2,0/1 et squalène/α-tocophérol supérieur à 10/1.

12. Extrait selon la revendication 10 ou la revendication 11, dans lequel l'extrait comprend un mélange d'environ 0,24 à 1,65 Mg/g d'hydroxytyrosol, d'environ 0,10 à 0,14 Mg/g de tyrosol, d'environ 0,05 à 3,5 mg d'oleuropéine, d'environ 0,01 à 0,08 mg/g d'a-tocophérol et d'environ 0,06 à 0,753 mg/g de squalène, par poids sec du matériau initial de marc.

13. Extrait selon l'une quelconque des revendications 10 à 12, dans lequel l'extrait est un extrait brut ou un extrait purifié, ou dans lequel l'extrait est un extrait en poudre ou un extrait liquide.

14. Formulation comprenant un extrait selon l'une quelconque des revendications 10 à 13, qui est un comprimé, une capsule, une capsule à enveloppe molle, un élixir, un sirop, une crème, une lotion, un gel ou une pommade.

15. Formulation selon la revendication 14, dans laquelle la formulation est une formulation pharmaceutique, une formulation de produit de santé complémentaire, une formulation cosmétique ou une formulation de produit alimentaire.
